# EUROPEAN PATENT SPECIFICATION

(11) **EP 1 641 747 B1**
(45) Date of publication and mention of the grant of the patent: **04.08.2010**
(21) Application number: 02785250.8
(22) Date of filing: 18.10.2002
(51) Int. Cl.: C07C 281/06, C09D 5/04

(54) **ACICULAR CRYSTALS OF HYDRAZINE-BASED DIUREA DERIVATIVES AND THEIR USE AS RHEOLOGY MODIFIERS IN COATING AND ADHESIVE COMPOSITIONS**
NADELFÖRMIGE KRISTALLE VON DIHARNSTOFFDERIVATEN DIE SICH VON HYDRAZIN ABLEITEN UND IHRE VERWENDUNG ZUR VERÄNDERUNG DER RHEOLOGY IN ÜBERZUGSZUSAMMENSETZUNGEN UND KLEBSTOFFEN
CRISTAUX ACICULAIRES DE DERIVES DE DIRUEE A BASE D'HYDRAZINE ET LEUR UTILISATION COMME MODIFICATEURS RHEOLOGIQUES DANS DES COMPOSITIONS DE REVETEMENT ET D'ADHESIFS

(30) Priority: 26.10.2001 EP 01204060
(43) Date of publication of application: 05.04.2006
(73) Proprietor: Nuplex Resins B.V., 4612 RB Bergen op Zoom (NL)
(72) Inventor: BRINKHUIS, Richard, Hendrikus, Gerrit, NL-8011 JX Zwolle (NL); ELFRINK, Petrus, Johannes, Maria, David, NL-5813 PZ Boxmeer (NL)
(74) Representative: Derks, Wilbert
(86) International application number: PCT/EP2002/011715
(87) International publication number: WO 2003/037849

(56) References cited:
- EP-A- 0 198 519
- WO-A-00/37520
- GB-A- 1 454 414
- JP-A- 2000 344 998
- US-A- 3 598 871
- US-A- 6 127 514
- CHEMICAL ABSTRACTS, vol. 67, no. 25, 18 December 1967 (1967-12-18) Columbus, Ohio, US; abstract no. 116858, M. FURDIK ET AL.: "Synthesis of azodicarboxylic acid imide, its N-substituted derivatives and their use as dienophiles in the Diesl-Alder reaction" page 11009; XP002190475 & CHEM. ZVESTI, vol. 21, no. 6, 1967, pages 427-42,
- DATABASE CROSSFIRE BEILSTEIN [Online] Beilstein Institut zur Förderung der Chemischen Wissenschaften, Frankfurt am Main, DE; Database accession no. Reaction ID 618514 and 1298566; and BRN 2660820 XP002190476 & H. GEHLEN ET AL.: JUSTUS LIEBIGS ANN. CHEM., vol. 685, 1965, pages 176-180,
- DATABASE CA [Online] CHEMICAL ABSTRACTS SERVICE, COLUMBUS, OHIO, US; TAKANO, TOSHURI ET AL.: "Thermal recording materials with specific urea compounds as color developers and reversible thermal recording method" retrieved from STN Database accession no. 123:325785 XP002231323 & JP 07 164757 A (NIPPON SEISHI KK) 27 June 1995 (1995-06-27)
- DATABASE CROSSFIRE BEILSTEIN [Online] Beilstein Institut zur Förderung der Chemischen Wissenschaften, Frankfurt am Main, DE; Database accession no. BRN 2390693 XP002190477 & KRAEBEL; DAVIS: J. CHEM. ENG. DATA, vol. 14, 1968, page 133, 134
- DATABASE CROSSFIRE BEILSTEIN [Online] Beilstein Institut zur Förderung der Chemischen Wissenschaften, Frankfurt am Main, DE; Database accession no. BRN 2771858 XP002190478 & SPECTROCHIM. ACTA PART A, vol. 23, 1967, pages 2541-49,
- DATABASE CROSSFIRE BEILSTEIN [Online] Beilstein Institut zur Förderung der Chemischen Wissenschaften, Frankfurt am Main, DE; Database accession no. BRN 2900497 XP002190479 & SU 384 331 A (DANILOV ET AL.) 5 December 1976 (1976-12-05)
- DATABASE CROSSFIRE BEILSTEIN [Online] Beilstein Institut zur Förderung der Chemischen Wissenschaften, Frankfurt am Main, DE; Database accession no. BRN 1717783 XP002190480 & FARMACO ED. SCI.,, vol. 10, 1955, page 619, 623
- DATABASE CROSSFIRE BEILSTEIN [Online] Beilstein Institut zur Förderung der Chemischen Wissenschaften, Frankfurt am Main, DE; Database accession no. BRN 2372381 XP002190560 & T. P. JOHNSTON, P. S. OPLIGER: J. MED. CHEM., vol. 10, 1967, pages 675-81,
- DATABASE CROSSFIRE BEILSTEIN [Online] Beilstein Institut zur Förderung der Chemischen Wissenschaften, Frankfurt am Main, DE; Database accession no. BRN 1807392 XP002190561 & TSUJI: PHARM. BULL., vol. 2, 1954, page 403, 411
- DATABASE CROSSFIRE BEILSTEIN [Online] Beilstein Institut zur Förderung der Chemischen Wissenschaften, Frankfurt am Main, DE; Database accession no. BRN 3445547 XP002190562 & MISTRY; GUHA: J. INDIAN INST. SCI. SECT., vol. 15, 1932, page 25, 35
- DATABASE CROSSFIRE BEILSTEIN [Online] Beilstein Institut zur Förderung der Chemischen Wissenschaften, Frankfurt am Main, DE; Database accession no. BRN 3191828 XP002190563 & SCHLOEGL: NATURWISSENSCHAFTEN, vol. 44, 1957, page 466
- DATABASE CROSSFIRE BEILSTEIN [Online] Beilstein Institut zur Förderung der Chemischen Wissenschaften, Frankfurt am Main, DE; Database accession no. BRN 2255203 XP002190564 & BUDESINSKY, Z. ET AL.: CESK. FARM., vol. 23, 1974, pages 200-206,
- DATABASE WPI Section Ch, Week 200025 Derwent Publications Ltd., London, GB; Class A21, AN 2000-286837 XP002190481 & JP 2000 080246 A (OTSUKA KAGAKU YAKUHIN KK), 21 March 2000 (2000-03-21)
- PATENT ABSTRACTS OF JAPAN vol. 011, no. 008 (C-396), 9 January 1987 (1987-01-09) & JP 61 185577 A (TOYO LINOLEUM MFG CO LTD:THE), 19 August 1986 (1986-08-19)
- PATENT ABSTRACTS OF JAPAN vol. 014, no. 227 (C-0718), 15 May 1990 (1990-05-15) & JP 02 055255 A (TOKUYAMA SODA CO LTD), 23 February 1990 (1990-02-23)
- DATABASE WPI Section Ch, Week 199528 Derwent Publications Ltd., London, GB; Class A60, AN 1995-213205 XP002231324 & JP 07 126588 A (CEMEDINE CO LTD), 16 May 1995 (1995-05-16)

## Description

The invention pertains to essentially formaldehyde-free coating and adhesive compositions, and thixotropic resin compositions, compositions comprising a hydrazine-based diurea derivative, and the use of the hydrazine-based diurea derivative as a rheology modifier and anti-settling agent.

Thixotropic agents or sag control agents are essential elements of coating and adhesive compositions. They not only provide a stable mixture of the several components in a coating composition, but also enable the application of the coating or adhesive, even in thick layers on vertically placed objects, without the occurrence of sags in the cured coating. Colloidal associates of urea compounds are known to be useful in that they provide a thixotropic rheology, improving, e.g., the sag resistance of coatings (hence the term sag control agent, SCA) or the orientation of metallic pigments in a base coat for automotive coatings and improving the appearance of coatings applied on vertical surfaces. Amine-based diurea derivatives are well-known sag control agents and are described in numerous patents. Known amine-based diurea derivatives are amino-functional compounds based on primary amines such as benzylamine, methylamine, hexylamine, 1,6-hexamethylenediamine, neopentyldiamine, and the like.

US3,598,871 discusses preparation of 1,6-dialkylbiureas by the reaction of hydrazine in a solvent with 2.0 to 5.0 moles of a corresponding 1,3-dialkylurea in the presence of from 0.05 to 14.0 moles of water and from 0.03 to 1.2 moles of an acid, by heating the solution in the range of 100°C to 170°C.

EP-A-0 198 519 discloses diurea compounds, the adducts of hexamethylene-1,6-diisocyanate (HDI) to benzylamine (BA), which are used in industry for the above purposes and are characterized by an anisotropic particle shape. The anisotropy is considered to allow for the formation of a percolating network of SCA particles upon flocculation (leading to a strong viscosity increase) at lower volume fractions than would be the case with isotropic particles. SEM pictures of HDI-BA diurea adducts reveal that the ratio of the longest to the shortest dimension (aspect ratio) is typically around five. The average length of these HDI-BA particles is 2 - 3 microns.

There have been substantial efforts to develop other compounds that can function as an SCA in coating (paint) and adhesive compositions.

We have found that specific alternative diurea compounds, namely those prepared from hydrazine (H₂N-NH₂) and mono-isocyanates, can be produced such that needle-like self-associated particles are formed that can or may show extreme anisotropy (aspect ratios over 300 in some cases). These needle-like particles were found to be more effective in inducing thixotropic rheology in coating formulations, at very low concentrations, than the existing di, tri and polyurea SCAs. The diurea compounds according to the invention can be used in resins for coatings with an improved appearance on vertical surfaces. They exhibit a broader application window than the known SCAs. In this context the term "application window" means the limits of layer thicknesses between which a coating or adhesive can be applied onto a vertical surface without sagging and with sufficient leveling for automotive and adhesive applications.

It is noted that the chemical compounds as such, obtained by, for example, reacting a mono-isocyanate and hydrazine, are well-known in the art. These products are known to be highly efficient scavengers of formaldehyde, for which purpose they have typically been used. The hydrazine-based diurea derivatives are consumed by said scavenging action. The physical form of the hydrazine-based diurea derivative is unimportant in this application.

According to the present invention there is provided a coating or adhesive composition, or thixotropic resin composition, according to claim 1.

According to another aspect of the invention there is provided the use of crystals as rheology modifiers in accordance with claim 5.

According to still further aspects of the invention there are provided a process to make a thixotropic resin according to claim 8; and a process to cure a coating or adhesive composition according to claim 11.

The present invention relates to specific compositions comprising hydrazine-based diurea derivatives of the formula R'HN-CO-NH-NH-CO-NHR, wherein R and R' independently are unsubstituted or substituted hydrocarbyl groups. These compositions are **characterized in that** they comprise the hydrazine-based diurea derivative in the form of acicular crystals, so that they function as an SCA, and in that the compositions are essentially free of formaldehyde or compounds that generate formaldehyde.

Preferably, the hydrocarbyl group is substituted or unsubstituted and selected from an alkyl, cycloalkyl, aralkyl, and aryl group. More preferably it is a substituted or unsubstituted monovalent aliphatic hydrocarbon group having 1 to 18 carbon atoms, a monovalent cycloaliphatic hydrocarbon group having 5 to 15 carbon atoms, a monovalent araliphatic hydrocarbon group having 7 to 15 carbon atoms, or a monovalent aromatic hydrocarbon group having 6 to 15 carbon atoms. Even more preferably, it is selected from methyl, butyl, hexyl, octadecyl, benzyl, methoxybenzyl, phenyl, methoxyphenyl, naphthalene, biphenyl, methoxypropyl, ethoxypropyl, methacryloyl-C₁₋₈ alkyl, preferably methacryloyl-C₁₋₄ alkyl, most preferably methacryloylethyl, with the formula 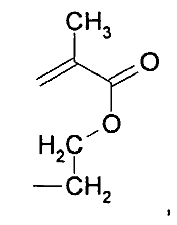 acryloyl-C₁₋₈ alkyl, preferably acryloyl-C₁₋₄ alkyl, most preferably acryloylethyl and mixtures thereof. Most preferred are the hydrocarbyl groups selected from hexyl, octadecyl, methoxypropyl, ethoxypropyl, benzyl, cyclohexyl, 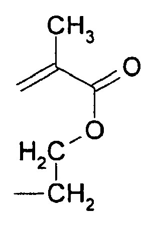 and combinations thereoflt is also preferred that R and R' are the same. Of these hydrazine-based SCAs, the compounds prepared by reacting hydrazine and cyclohexyl isocyanate, octadecyl isocyanate, or a mixture thereof, the hydrocarbyl groups therefore being cyclohexyl, octadecyl, and mixtures thereof, are especially preferred.

The acicular form of the crystals can be characterized by the aspect ratio, i.e. the length (longest dimension) of the crystals divided by their width, as can be determined by (visual) inspection of SEM pictures. This aspect ratio should be more than 3, preferably more than 10, most preferably more than 20. Another important characteristic is the average length (le) of the hydrazine-based diurea derivative particles, as can be determined by (visual) inspection of SEM pictures. Depending on the conditions chosen when making the hydrazine-based diurea derivative, the le can vary from 0.1 to 300 micrometres (microns). Suitably the conditions are chosen such that the le is more than 0.5, preferably more than 1, more preferably more than 2, and most preferably more than 3 microns. The lower numbers are less preferred, since compositions comprising the hydrazine-based diurea derivative with such an le may still suffer from sagging or may require high loads of the SCA. In order to control the viscosity of compositions in which the SCA is used, the le preferably is less than 100, more preferably less than 50, and most preferably less than 30 microns. If the viscosity of a composition comprising a hydrazine-based diurea derivative according to the invention is too high, the le can be reduced or the amount of the hydrazine-based diurea derivative can be decreased, and *vice versa*.

The term "hydrazine" means hydrazine, hydrazine hydrate or salts thereof. Preferably, aqueous solutions of hydrazine are used.

The term "mono-isocyanate-functional compounds" is used to denominate isocyanates that contain only one amine-reactive isocyanate group per molecule.

The term "essentially free of formaldehyde or compounds that generate formaldehyde" is used here to denote compositions that do not contain or generate enough formaldehyde during regular processing to completely consume the SCA. Preferably, the compositions are completely free of formaldehyde during their processing, because then no SCA will be consumed before the final coating or adhesive composition is applied or formed, ensuring the full sag control effect of the SCA that was introduced. However, for two-component coating or adhesive formulations it can be advantageous to use a small amount of a formaldehyde-based resin or a compound that generates formaldehyde in order to lower the curing temperature and to obtain a clear, haze-free coating in the case of clear coats. In that case, the amount of formaldehyde formed during processing and curing of the coating or adhesive compositions preferably is less than 100%, more preferably less than 50%, even more preferably less than 25% of the amount that can be scavenged by the hydrazine-based diurea derivative present in the formulation.

The formaldehyde-based resin or compound that generates formaldehyde during the curing conditions is preferably added just before application or it can be added to the component of the two-component coating which does not contain the hydrazine-based diurea derivative. The term "essentially acicular" is meant to denominate crystals with a length in one dimension that is at least 2 times, preferably 4 times, more preferably at least 6 times, and most preferably at least 8 times, the length in any other dimension. Typically the crystals are needle-shaped.

The term "adhesive" is meant to denominate cements, glues, sealants, as well as putties.

Conventional sag control agents are known to have been produced in the presence of a binder resin. In GB 1,454,414, for example, a urea adduct is prepared *in situ* in the presence of the binder. Also the hydrazine-based diurea derivatives according to the invention are preferably produced in the presence of a binder so as to directly form needles of the desired le in the binder resin. However, they can also be produced by reacting hydrazine and isocyanate in a non-solvent with a limited solubility for the hydrazine-based diurea derivative, resulting in a precipitate that can be used as such, be milled in order to reduced the le, or be recrystallized, e.g., to increase the purity or to change the le. In the process of making the precipitate of the hydrazine-based diurea derivative the dosing conditions can be varied and the stirrer speed changed in order to change the le of the resulting needles. Such optimizations are known to the skilled person.

Alternatively, the hydrazine-based diurea derivative can be prepared as a true solution at temperatures between 0 and 150°C. The needles of the hydrazine-based diurea derivative can subsequently be precipitated from the solution using conventional techniques, such as the addition of or addition to a non-solvent, and then mixed with the binder. However, if the solvent is selected such that it has affinity for the binder, the solution can be combined with said binder and form the precipitate directly in said binder. Suitable solvents for this purpose are, for example, N-methyl pyrrolidone (NMP) or butylglycol.

These known methods are equally applicable with the present invention. Usually, hydrazine or a hydrate or a salt thereof is reacted with at least one mono-isocyanate of the formula R-NCO or R'-NCO, wherein R and R' have the previously given meanings, in one of the above-disclosed manners. Also mixtures of mono-isocyanates of formulae R-NCO or R'-NCO can be used. The reaction between the hydrazine and the mono-isocyanate-functional compound can generally be carried out in any arbitrary sequence of combining the starting compounds, optionally at elevated or lowered temperature. It is preferred that the reaction should be carried out in an atmosphere of an inert gas at temperatures in the range of -20 to +80°C. The reaction can be performed with mixtures of mono-isocyanates R-NCO and R'-NCO, wherein R and R' have a different meaning, or with just one isocyanate of the formula R-NCO.

If the overall amount of hydrazine and the isocyanate-functional compound is stoichiometric, then each amine group reacts with an isocyanate group of the isocyanate-functional compound, such that essentially no free amine or isocyanate groups are present after completion of the theoretical reaction. However, in order to prevent the presence of small amounts of unreacted amino groups of the hydrazine, a slight excess of isocyanate-functional compound is preferred. Preferably, this excess ranges from 1 to 10%, based on equivalents.

In accordance with a preferred embodiment, the thixotropic resin with the hydrazine-based diurea derivative is obtainable by adding a mono-isocyanate-functional compound to a mixture of hydrazine and resin, resulting in anisotropic acicular crystals embedded in the resin. However, the hydrazine and the mono-isocyanate-functional compound can be added to the resin in any sequence. If so desired, this may be done in several steps. If the mono-isocyanate-functional compound is added first, care should be taken that the thus obtained mixture is not stored for too long or at a too high temperature, since a reaction between the resin and the mono-isocyanate-functional compound might occur. It is also possible to add the amine compound to a portion of the resin and to add the isocyanate-functional compound to another portion of the resin, followed by combining the two resin mixtures, optionally with additional resin being added. Alternatively, the hydrazine-based diurea derivative can be made in a coating formulation instead of in a mere resin using any sequence described above.

During the reaction of the amine and the isocyanate-functional compounds the reaction mixture is preferably thoroughly mixed. Suitably this is done by using low- or high-shear mixers (such as propellers, impellors, turbines, rotor-stator mixers, dispersers, dissolvers, and the like).

Additionally, if a precipitate is formed, it can be preferable that one or more additives which function as a crystal growth modifier and/or flocculation modifier for the SCA particles are present.

When crystal growth modifiers are used, the le of the SCA needles can be influenced. One or more crystal growth modifiers can be added before or simultaneously with the formation of the hydrazine-based diurea derivative. Alternatively, one or more of the crystal growth modifiers are formed during the crystallization step of the hydrazine-based diurea derivative. Preferably, the crystal growth modifiers comprise at least one polar, preferably "ureaphilic," i.e. multiple hydrogen bridging group, with the proviso that the crystal growth modifiers are different from the main crystallizing diurea compound. Such molecules will interact with the hydrazine-based diurea derivative that is formed, retarding further crystallization of the nucleus. Preferred ureaphilic groups are urea, amide, and urethane groups. Whether or not a compound with ureaphilic groups is an effective crystal growth modifier is easily determined by carrying out the precipitation step of the hydrazine-based diurea derivative both with and without 2% by weight of said component (based on the hydrazine-based diurea derivative) and to judge the differences in aspect ratio and/or length of the needles that are formed.

Flocculation modifiers can be added before, simulataneously with, or after the formation of the hydrazine based diurea derivative. When flocculation modifiers are used, the stabilization of the SCA crystals towards flocculation can be enhanced, and flocculation retarded. Such additives are characterized by a molecular weight of at least 500 Dalton, more preferably, the additives are selected from molecules with a molecular weight of at least 600 Dalton, even more preferably at least 800 Dalton, more preferably still at least 1,000 Dalton ,and most preferably more than 2,000 Dalton, and comprise a polar (preferably ureaphilic) moiety as described above, as well as a moiety, such as a main chain, that has good compatibility with the resin/solvent in which the precipitate is formed. Such compounds may have both a crystal growth modifier as well as a flocculation modifier function. Flocculation retardation can be observed as a (relative) slowing down of the structure build-up after high shear treatment in a rheological creep test. Suitable modifiers are molecules with one or more urea groups such as are for example obtainable by reacting an isocyanate with a bulky or long-chain amine, such as Jeffamine M1000.

The preparation of the hydrazine-based diurea derivative can be performed batchwise as well as in a continuous process.

The amount of SCA to be used in a binder concentrate, a coating composition or an adhesive composition can vary over a wide range, depending on the circumstances. In a preferred embodiment, the SCA according to the invention is present in a concentrated resin composition. Such concentrates have particular advantages when making the coating or adhesive compositions. More specifically, they ensure great flexibility and dosing accuracy of the SCA. At present it is expected that the concentrates will contain from 0.1 to 50% by weight of the SCA, based on the weight of the total concentrate (%w/w). Preferably, the amount of SCA in the concentrate is greater than 0.1, more preferably greater than 0.5, even more preferably greater than 1.0, and most preferably greater than 1.5 %w/w. It is preferred that the concentrate comprises less than 50, more preferably less than 25, and most preferably less than 10 %w/w of the SCA. Especially since these concentrates are typically stored for a prolonged period of time before they are used in making the coating or adhesive formulation, it is preferred that they are formaldehyde-free. The amount of SCA present in the coating or adhesive formulation depends, *inter alia*, on the rheology of the formulation in which it is used and the sag control required. It is noted that an SCA according to the invention can be combined with other rheology control agents such as conventional SCA based on HDI-BA such as described, for example, in EP-A-0 192 304. Especially when the curing temperature of the final coating or adhesive is over 100°C, such a combination with conventional SCAs can be advantageous. Typically, the total SCA content originating from both types of SCA is between 0.1 and 5 %. Alternatively, the hydrazine-based diurea derivative can be combined with nonaqueous dispersions or microgels in coating formulations in order to optimize the rheological behaviour and performance of coatings.

The thixotropic resins according to the present invention are pre-eminently suited to be used in the formulation of coating and adhesive compositions. In coating compositions these resins can be used as a functional binder in combination with cross-linkers, such as cross-linking resins, e.g., for curing in 1 K- and 2K-coating compositions at room temperature, forced drying conditions (30-100°C), and baking conditions (higher than 100°C). Forced drying and baking conditions are preferred. High-temperature baking is preferably performed at 100-160°C. Furthermore, the present rheology modifiers have excellent sag resistance at lower concentrations than the known rheology modifiers, such as SCAs based on di-, tri-, or polyisocyanates with monoamines.

It should be noted that the cross-linker used in the final coating or adhesive formulation can be of any conventional type suitable for the resin used, as long as the formulation is essentially formaldehyde-free. Known resin/cross-linker and/or cross-linker/resin combinations in which the SCA according to the invention can be used include: polyol or thiol/(blocked) polyisocyanate; epoxy/acid combinations; epoxy/anhydride combinations; epoxy/acid-anhydride; acetoacetate/amine, aldehyde blocked amine, or ketone blocked amine; acryloyl/amine, aldehyde blocked amine, or ketone blocked amine; epoxy/thiol. Polyols include acrylic polyols, polyester polyols, polyether polyols, silicon-containing polyols, urethane modified polyols and (internally) blocked polyols, and mixtures thereof.

However, the SCA according to the invention can also be used in compositions where curing is obtained by means of actinic radiation, such as near-infra red, visible light, UV and X-ray radiation or electron beam radiation. Such curing processes can involve mere curing through unsaturated covalent bonds, such as ethylenically unsaturated carbon-carbon bonds, but any other conventional reactive bond may be involved in the curing process as well. Also, it can be beneficial to use a conventional radiation-susceptible activator/initiator in such curing processes. Examples of suitable radiation-curable compositions are presented in WO 02/18468. The conventional SCA as used therein can be replaced by the SCA according to the invention.

It was found that any resin common in coatings and adhesives can be used in the present invention. Examples are polyesters, ester diols, polyurethanes, alkyd resins, acrylate and methacrylate resins, epoxy resins, polyamine, carbamate, and polyester acrylate resins, and the like. These resins may optionally be functionalized with hydroxy, keto, carboxylate, sulfonate, carbamate, epoxy groups, tertiary amines, and the like. Also, the final coating or adhesive composition can be solvent or water borne.

The thixotropic coating composition can further contain the usual adjuvants and additives, for instance pigment dispersants, dyes, pigments, solvents, and accelerators for the curing reaction.

The thixotropic coating composition can be applied to a substrate in any desired manner, for instance by rolling, spraying, brushing, sprinkling, casting, dipping or electrostatic spraying. The thixotropic coating composition can further be cured or baked in the usual way, for instance at ambient temperatures or in an oven at higher temperatures, for instance in the range of 60 to 140°C, over a period of 2 to 120 minutes.

The invention is further illustrated by the following examples:

### Example 1

2.96 g of a hydrazine solution (35 wt.% in water) were added to 300 g of a polyester polyol resin (Setal® 166 SS-80, 80% solids in butyl acetate) at room temperature. Under stirring, a solution of 8.08 g of cyclohexyl isocyanate (CHI) was added over a period of 2 min. Stirring was continued for 5 - 10 min. A turbid colloidal suspension with needles of the reaction product (HY/CHI) of hydrazine (HY) and cyclohexyl isocyanate (CHI) having an le of about 60 - 70 microns with strong thixotropy was obtained.

### Example 2

Into a 2-litre reaction vessel equipped with a high-speed dissolver and two dropping funnels were charged 600 g of Setal® 166 SS-80 (a polyester polyol resin, ex Akzo Nobel) and 138.5 g of butyl acetate. The contents of the reaction vessel were mixed at 1,700 rpm. Then 5.84 g of a 35% aqueous hydrazine solution were added and mixed at 1,700 rpm for 5 minutes. Next a mixture of 16.44 g of cyclohexylisocyanate and 16.44 g of butyl acetate was added in 6 min at 3,000 - 3,550 rpm. After the addition, the reaction mixture was stirred for another 10 min at 4,000 - 4,350 rpm.

The obtained white solution had a theoretical percentage of 2.32 of the hydrazine-based diurea compound. The viscosity at 1 and 100 s⁻¹ amounted to 14.1 and 0.64 Pas, respectively.

### Example 3

6.23 g of an adduct of Jeffamine® M1000 to cyclohexylisocyanate (CHI) (1:1 molar), which acts as a crystal growth inhibitor, were added to 150 g Setal® 166 solution (80% solids); 1.47 g of hydrazine (35 wt.% aqueous solution) were added, and under stirring 4.02 g of CHI were added over 2 min. Stirring was continued for 5 - 10 min, and a turbid colloidal suspension with strong thixotropic rheology was obtained.

SEM analysis of the colloidal particles formed in the resin revealed the extreme aspect ratio (long thin needles).

### Example 4

138.46 g of an adduct of Jeffamine® M1000 to CHI (1:1 molar) were added to 600 g of a polyester polyol resin (Setal® 166, 80% solids) at room temperature followed by the addition of 5.84 g of hydrazine solution (35 wt.% in water). Under stirring, a solution of 16.44 g of CHI in 16.44 g of butyl acetate was added over a period of 6 min. Stirring was continued for 10 min. A turbid colloidal suspension with strong thixotropy was obtained. The fineness of the urea suspension was shown to be <8 µm (using a Hegman bar).

### Example 5

0.71 g of a hydrazine solution (35 wt.% in water) was added to 150 g of a polyester polyol resin (Setal® 166, 80% solids) at room temperature. Under stirring, a solution of 4.27 g of octadecyl isocanate (ODI) was added over a period of <2 min. Stirring was continued for 5 - 10 min. A thixotropic turbid colloidal suspension containing needles was obtained.

### Example 6

Into a 2-litre reaction vessel equipped with a high-speed dissolver and two dropping funnels were charged 400 g of Setal® 166 SS-80 (a polyester polyol resin, ex Akzo Nobel) and 92.3 g of butyl acetate. The contents of the reaction vessel were mixed at 1,700 rpm. Then 3.89 g of a 35% aqueous hydrazine solution were added and mixed at 1,700 rpm for 5 minutes. Next a mixture of 10.96 g of cyclohexyl isocyanate and 10.96 g of butyl acetate was added in 6 min at 4,500 - 4,800 rpm. After the addition, the reaction mixture was stirred for another 20 min at 14,000 rpm.

The obtained white solution had a theoretical percentage of 2.32 of the hydrazine-based diurea compound. The viscosity at 1 and 100 s⁻¹ amounted to 15.5 and 0.66 Pa.s, respectively. The needles had an le of 10 to 50 microns.

### Example 7

A lacquer was formulated by adding 1.38 g of the product of Example 3 to 3.15 g of Setal® 166 (80% solids). Stirring was continued until a homogeneous mixture was obtained. Under stirring, 1.12 g of butyl acetate were added slowly over a period of 510 min. To this homogeneous mixture 2.29 g of Tolonate® HDT-LV were added (total 0.67 wt.% diurea on solids, solids content 75%). Stirring was continued for 5 min. The rheology of the lacquer was measured and showed a fast recovery over more than two decades (75 Pa.s after 200 sec at 0.5 Pa shear stress after disturbing at 1,000 s⁻¹; high-shear viscosity 0.5 Pa.s.

The lacquer was applied to a glass substrate with a doctor blade at a film thickness of 120 µm, and cured for 30 min at 90°C. A clear glossy film was obtained: no turbidity of colloidal particles was visible.

### Example 8

In a first series two paints A (according to the invention) and B (comparative) were prepared according to Table I. The paints were sprayed onto a vertical tin plate with 13 holes of 1 cm diameter and vertically baked at the indicated schedules. The sagging limit was determined as the layer thickness between the hole at which sagging occurs and the preceding hole. Another way to measure sagging is to measure the layer thickness at the point where the length of the tear is 1 cm. The resin of Example 2 is more effective than Setal® 80166 SS-64. The higher the sagging limit, the more effective the SCA is.

**Table I**

| | A | B |
|---|---|---|
| Setal® 166 SS-80 (solids) | 43.0 | 43.0 |
| Resin of Example 2 (solids) | 18.2 | - |
| Setal® 80166 SS-64 (solids) | - | 18.2 |
| Silicon oil L050 (1 % in butyl acetate) | 5.0 | 5.0 |
| Tinuvin® 292 | 0.5 | 0.5 |
| Tinuvin® 1130 | 1.0 | 1.0 |
| Tinstab® BL 277 (1 % in butyl acetate) | 0.7 | 0.7 |
| Tolonate® HDT 90 (solids) | 38.8 | 38.8 |
| Butyl acetate to 23 sec DIN cup 4/23°C | | |
| % SCA | 0.68 | 0.68 |
| Forced drying for 45 min at 80°C | | |
| Sagging limit (micron) | 61 | 48 |
| Tear 1 cm (micron) | 88 | 68 |
| Baking for 24 min at 140°C | | |
| Sagging limit (micron) | 53 | <41 |
| Tear 1 cm (micron) | 71 | 54 |

### Example 9

In a second series (see Table II) again two paints C (according to the invention) and D (comparative) were prepared and applied. The sagging limit was measured as described in Example 8. Thus the effectiveness as anti-sagging agent at low SCA concentration becomes clear.

**Table II**

| | C | D |
|---|---|---|
| Setal® 166 SS-80 (solids) | 43.0 | 61.2 |
| Resin of Example 2 (solids) | 18.2 | - |
| Setal® 80166 SS-64 (solids) | - | - |
| Silicon oil L050 (1 % in butyl acetate) | 5.0 | 5.0 |
| Tinuvin® 292 | 0.5 | 0.5 |
| Tinuvin® 1130 | 1.0 | 1.0 |
| Tinstab® BL 277 (1 % in butyl acetate) | 0.7 | 0.7 |
| Tolonate® HDT 90 (solids) | 38.8 | 38.8 |
| Butyl acetate to 23 sec DIN cup 4/23°C | | |
| % SCA | 0.68 | 0.0 |
| Forced drying for 45 min at 80°C | | |
| Sagging limit (micron) | 56 | 28 |
| Tear 1 cm (micron) | 69 | 46 |
| Baking for 24 min at 140°C | | |
| Sagging limit (micron) | 43 | 26 |
| Tear 1 cm | 58 | 43 |

### Comparative Example 10

162.1 g of Setal® 80166 (64% solids, 3.8% SCA ("clear" triurea type on solids) were added to 306.3 g of Setal® 166 (80% solids). Stirring was continued until a homogeneous mixture was obtained. Under stirring 72.15 g of butyl acetate were added slowly over a period of 5 - 10 min. Finally, 221.04 g of Tolonate® HDT-LV were added (solids content 75%, SCA on solids 0.68%). Stirring was continued for 5 min. The rheology was measured and compared with that of the lacquer from Example 6. At this concentration a very limited structural viscosity was built up after high shear disturbance (0.5 Pa.s after 200 sec at 0.5 Pa shear stress after disturbing at 1,000 s⁻¹; high-shear viscosity 0.5 Pa.s).

### Comparative Example 11

Into a 2-litre reaction vessel equipped with a high-speed dissolver and two dropping funnels were charged 600 g of Setal® 166 SS-80 and 131.6 g of butyl acetate. The contents of the reaction vessel were mixed at 1,700 rpm. Then 10.08 g of benzylamine were added and mixed at 1,700 rpm for 5 minutes. Next a mixture of 8.15 g of benzylamine and 16.31 g of butyl acetate was added in 6 minutes at 3,000 - 4,500 rpm. After the addition, the reaction mixture was stirred for another 10 minutes at 3,800 - 4,000 rpm.

The obtained white solution had a theoretical percentage of 2.32 of HDI/BA SCA. The viscosity at 1 and 100 s⁻¹ amounted to 4.55 and 0.51 Pas, respectively.

### Example 12

The following clear coat formulations were prepared. The clear coats were formulated in such a way that on solids all formulations contained the same amounts of acrylic resin and polyester resin. Examples A-C are comparative.

**Table III**

| Formulation | A | B | C | D | E | F |
|---|---|---|---|---|---|---|
| Setalux® 1767 VV-65 (solids) | 34.1 | 34.1 | 34.1 | 34.1 | 34.1 | 34.1 |
| Setal® 166 SS-80 (solids) | 10.7 | 5.3 | | 10.7 | 5.3 | |
| Resin of example 11 - Setal® 166 SS-80 modified with HDI/BA (solids) | 18.8 | 24.2 | 29.5 | | | |
| Setal® 166 SS-80 modified with 3.75% (on solids) of HY/CHI (solids) 1 | | | | 18.8 | 24.2 | 29.5 |
| Tinstab® BL 277 (1% in BuAc) | 0.9 | 0.9 | 0.9 | 0.9 | 0.9 | 0.9 |
| Baysilon® OL-17 (1% in BuAc) | 3.0 | 3.0 | 3.0 | 3.0 | 3.0 | 3.0 |
| BYK® 306 | 0.5 | 0.5 | 0.5 | 0.5 | 0.5 | 0.5 |
| Tinuvin® 292 | 0.5 | 0.5 | 0.5 | 0.5 | 0.5 | 0.5 |
| Tinuvin® 1130 | 1.0 | 1.0 | 1.0 | 1.0 | 1.0 | 1.0 |
| Tolonate® HDT 90 (solids) | 36.4 | 36.4 | 36.4 | 36.4 | 36.4 | 36.4 |
| Butyl acetate | 3.4 | 1.7 | | 3.4 | 1.7 | |
| Thinner to 28 s DIN 4, 23 °C; Solvesso® 100/methoxy Propylacetate (1:1) °C; | .. | .. | .. | .. | .. | .. |
| | | | | | | |
| Theoretical SCA content (%) | 0.7 | 0.9 | 1.1 | 0.7 | 0.9 | 1.1 |

| | | | | | | |
|---|---|---|---|---|---|---|
| ¹ The average length (as determined with Scanning Electron Microscopy) of the needles of the reaction product of hydrazine and cyclohexyl isocyanate of Setal® 166 SS-80 modified with 3.75% (on solids) of HY/CHI used for the determination of the appearance as described above amounted to 3 - 5 microns. | | | | | | |

Tin plated steel panels of 30x50 cm were coated with a commercial solvent borne primer having an average layer thickness of 40 microns and a water borne pepper red base coat having an average layer thickness of 12-15 microns using a spray robot.

The clear coats were applied with high-speed bell spraying equipment in three different layer thicknesses (20, 30, and 40 microns). During spraying, flash-off, and baking (24 min. at 140 °C) of the clear coats the panels were vertically positioned.

The appearance of the clear coats was measured using a commercially available wave scan apparatus and was expressed as GM-tension. The results can be found in the following table.

**Table IV GM-Tension**

| Formulation | A | B | C | D | E | F |
|---|---|---|---|---|---|---|
| Type of SCA | HDI/BA | HDI/BA | HDI/BA | HY/CHI | HY/CHI | HY/CHI |
| Theoretical % of SCA | 0.7 | 0.9 | 1.1 | 0.7 | 0.9 | 1.1 |
| Layer thickness 20 micron | 7.5 | 8.2 | 7.7 | 7.7 | 8.6 | 8.3 |
| Layer thickness 40 micron | 11.3 | 12.2 | 10.8 | 14.4 | 12.7 | 11.9 |

As can be judged from Table IV, the appearance defined in terms of tension of the clear coats formulated with the HY/CHI SCA has improved in comparison with the HDI/BA SCA at both 20 and 40 microns.

## Claims

1. A coating or adhesive composition, or a thixotropic resin composition, comprising crystals having a length in one dimension that is at least two times the length in any other dimension, of a hydrazine-based diurea derivative rheology modifier of the formula
R'HN-CO-NH-NH-CO-NHR
wherein R and R' independently are unsubstituted or substituted hydrocarbyl groups, with an average length of the crystals of 0.1 to 300 micrometres, the composition being essentially free of formaldehyde or compounds that generate formaldehyde, to the extent that there is not enough formaldehyde to completely consume the hydrazine-based diurea derivative.

2. A coating or adhesive composition according to claim 1 comprising a hydrazine-based diurea derivative wherein R and R' are the same.

3. A coating or adhesive composition according to claim 1 or 2 comprising a hydrazine-based diurea derivative wherein the hydrocarbyl group is selected from an alkyl, cycloalkyl, aralkyl, and aryl group.

4. A coating or adhesive composition according to claim 3 wherein the hydrocarbyl group is selected from methyl, butyl, hexyl, octadecyl, benzyl, methoxybenzyl, phenyl, methoxyphenyl, naphthalene, biphenyl, methoxypropyl, ethoxypropyl, methacryloylethyl, and acryloylethyl, whereof hexyl, octadecyl, benzyl, and cyclohexyl are most preferred.

5. Use of crystals having a length in one dimension that is at least two times the length in any other direction, of a hydrazine-based diurea derivative of the formula
R'HN-CO-NH-NH-CO-NHR
wherein R and R' independently are unsubstituted or substituted hydrocarbyl groups, with an average length of the crystals of 0.1 to 300 micrometres, as a rheology modifier.

6. The thixotropic resin composition according to claim 1 wherein the hydrazine-based diurea derivative is present in a concentrated amount of 0.1 to 50% by weight, based on the weight of the composition.

7. Use of a resin according to claim 5 in the process to make coating or adhesive compositions.

8. Process to make a thixotropic resin composition comprising a hydrazine-based diurea derivative rheology modifier, involving the steps of i) reacting at least one mono-isocyanate-functional compound of the formula R.NCO, wherein R has the meaning as defined in claims 1-4, with a hydrazine to form a hydrazine-based diurea derivative, and ii) combining the hydrazine-based diurea derivative with a resin, to obtain needle-like crystals of the hydrazine-based diurea derivative with an average length of from 0.1 to 300, which are embedded in the resin
the resin being essentially free of formaldehyde or compounds that generate formaldehyde, to the extent that there is not enough formaldehyde to completely consume the hydrazine-based diurea derivative.

9. A process in accordance with claim 8 wherein the isocyanate and the hydrazine are reacted in the presence of the resin such that the embedded needle-like crystals of the hydrazine-based diurea derivative are obtained in a single step.

10. A process according to claim 8 or 9 wherein one or more crystal growth modifiers are used to reduce the average length of the crystals to 0.1 to 100 micrometers.

11. A process to cure a coating or adhesive composition comprising crystals having a length in one dimension that is at least two times the length in any other direction, of a hydrazine-based diurea derivative rheology modifier of the formula
R'HN-CO-NH-NH-CO-NHR
wherein R and R' independently are unsubstituted or substituted hydrocarbyl groups, with an average length of the crystals of 0.1 to 300 micrometres, in which process forced drying and/or baking conditions are applied.

12. The use of claim 5 wherein the crystals of hydrazine-based diurea derivative are used as a sag control agent or an anti-setting agent in coating and/or adhesive compositions.

13. The process according to claim 8 wherein the average length of the needle-like crystals of the hydrazine based diurea derivative is from 0.5 to 100 micrometers.

## Patentansprüche

1. Beschichtungs- oder Kleberzusammensetzung oder thixotrope Harzzusammensetzung, umfassend Kristalle, die eine Länge in der einen Dimension aufweisen, welche wenigstens das Zweifache der Länge in jeder anderen Dimension beträgt, aus einem Rheologiemodifikator in Form eines auf Hydrazin basierenden Diharnstoff-Derivats der Formel R'HN-CO-NH-NH-CO-NHR,
wobei R und R' unabhängig voneinander unsubstituierte oder substituierte Hydrocarbylgruppen sind, mit einer mittleren Länge der Kristalle von 0,1 bis 300 Mikrometer, wobei die Zusammensetzung im Wesentlichen frei von Formaldehyd oder Verbindungen, die Formaldehyd erzeugen, ist, und zwar bis zu einem solchen Grad, dass nicht genug Formaldehyd vorhanden ist, um das auf Hydrazin basierende Diharnstoff-Derivat vollständig zu verbrauchen.

2. Beschichtungs- oder Kleberzusammensetzung gemäß Anspruch 1, die ein auf Hydrazin basierendes Diharnstoff-Derivat umfasst, bei dem R und R' gleich sind.

3. Beschichtungs- oder Kleberzusammensetzung gemäß Anspruch 1 oder 2, die ein auf Hydrazin basierendes Diharnstoff-Derivat umfasst, bei dem die Hydrocarbylgruppe aus einer Alkyl-, Cycloalkyl-, Aralkyl- und Arylgruppe ausgewählt ist.

4. Beschichtungs- oder Kleberzusammensetzung gemäß Anspruch 3, wobei die Hydrocarbylgruppe aus Methyl, Butyl, Hexyl, Octadecyl, Benzyl, Methoxybenzyl, Phenyl, Methoxyphenyl, Naphthalin, Biphenyl, Methoxypropyl, Ethoxypropyl, Methacryloylethyl und Acryloylethyl ausgewählt ist, wovon Hexyl, Octadecyl, Benzyl und Cyclohexyl am meisten bevorzugt sind.

5. Verwendung von Kristallen, die eine Länge in der einen Dimension aufweisen, die wenigstens das Zweifache der Länge in jeder anderen Dimension beträgt, aus einem auf Hydrazin basierenden Diharnstoff-Derivat der Formel
R'HN-CO-NH-NH-CO-NHR
wobei R und R' unabhängig voneinander unsubstituierte oder substituierte Hydrocarbylgruppen sind, mit einer mittleren Länge der Kristalle von 0,1 bis 300 Mikrometer, als Rheologiemodifikator.

6. Thixotrope Harzzusammensetzung gemäß Anspruch 1, wobei das auf Hydrazin basierende Diharnstoff-Derivat in einer konzentrierten Menge von 0,1 bis 50 Gew.-% vorhanden ist, bezogen auf das Gewicht der Zusammensetzung.

7. Verwendung eines Harzes gemäß Anspruch 5 bei dem Verfahren zur Herstellung von Beschichtungs- oder Kleberzusammensetzungen.

8. Verfahren zur Herstellung einer thixotropen Harzzusammensetzung, die einen Rheologiemodifikator in Form eines auf Hydrazin basierenden Diharnstoff-Derivats umfasst, umfassend die Schritte des i) Umsetzens wenigstens einer monoisocyanatfunktionellen Verbindung der Formel RNCO, wobei R die in den Ansprüchen 1 bis 4 definierte Bedeutung hat, mit einem Hydrazin unter Bildung eines auf Hydrazin basierenden Diharnstoff-Derivats und ii) Kombinieren des auf Hydrazin basierenden Diharnstoff-Derivats mit einem Harz, wobei man nadelartige Kristalle des auf Hydrazin basierenden Diharnstoff-Derivats mit einer mittleren Länge von 0,1 bis 300 µm erhält, die in das Harz eingebettet sind, wobei das Harz im Wesentlichen frei von Formaldehyd oder Verbindungen, die Formaldehyd erzeugen, ist, und zwar bis zu einem solchen Grad, dass nicht genug Formaldehyd vorhanden ist, um das auf Hydrazin basierende Diharnstoff-Derivat vollständig zu verbrauchen.

9. Verfahren gemäß Anspruch 8, wobei das Isocyanat und das Hydrazin in Gegenwart des Harzes umgesetzt werden, so dass die eingebetteten nadelartigen Kristalle des auf Hydrazin basierenden Diharnstoff-Derivats in einem einzigen Schritt erhalten werden.

10. Verfahren gemäß Anspruch 8 oder 9, wobei ein oder mehrere Kristallwachstumsmodifikatoren verwendet werden, um die mittlere Länge der Kristalle auf 0,1 bis 100 Mikrometer zu reduzieren.

11. Verfahren zur Härtung einer Beschichtungs- oder Kleberzusammensetzung, die Kristalle, welche eine Länge in der einen Dimension aufweisen, die wenigstens das Zweifache der Länge in jeder anderen Dimension beträgt, aus einem Rheologiemodifikator in Form eines auf Hydrazin basierenden Diharnstoff-Derivats der Formel
R'HN-CO-NH-NH-CO-NHR,
wobei R und R' unabhängig voneinander unsubstituierte oder substituierte Hydrocarbylgruppen sind, mit einer mittleren Länge der Kristalle von 0,1 bis 300 Mikrometer umfasst, wobei bei diesem Verfahren Bedingungen für ein Schnelltrocknen und/oder -brennen angewendet werden.

12. Verwendung gemäß Anspruch 5, wobei die Kristalle des auf Hydrazin basierenden Diharnstoff-Derivats als Antiabsackmittel oder Antiabsetzmittel in Beschichtungs- und/oder Kleberzusammensetzungen verwendet werden.

13. Verfahren gemäß Anspruch 8, wobei die mittlere Länge der nadelartigen Kristalle des auf Hydrazin basierenden Diharnstoff-Derivats 0,5 bis 100 Mikrometer beträgt.

## Revendications

1. Composition de revêtement ou adhésive, ou composition de résine thixotrope, comprenant des cristaux dont la longueur dans une dimension est au moins deux fois la longueur dans toute autre dimension, d'un modificateur de rhéologie dérivé de diurée à base d'hydrazine de formule
R'HN-CO-NH-NH-CO-NHR
dans laquelle R et R' sont indépendamment des groupes hydrocarbyle non substitués ou substitués, avec une longueur moyenne des cristaux de 0,1 à 300 micromètres, la composition étant essentiellement dépourvue de formaldéhyde ou de composés qui génèrent du formaldéhyde, dans la mesure où il n'y a pas assez de formaldéhyde pour consommer complètement le dérivé de diurée à base d'hydrazine.

2. Composition de revêtement ou adhésive selon la revendication 1, comprenant un dérivé de diurée à base d'hydrazine dans lequel R et R' sont identiques.

3. Composition de revêtement ou adhésive selon la revendication 1 ou 2, comprenant un dérivé de diurée à base d'hydrazine dans lequel le groupe hydrocarbyle est choisi parmi un groupe alkyle, cycloalkyle, aralkyle et aryle.

4. Composition de revêtement ou adhésive selon la revendication 3, dans laquelle le groupe hydrocarbyle est choisi parmi les groupes méthyle, butyle, hexyle, octadécyle, benzyle, méthoxybenzyle, phényle, méthoxyphényle, naphtalène, biphényle, méthoxypropyle, éthoxypropyle, méthacryloyléthyle et acryloyléthyle, où les groupes hexyle, octadécyle, benzyle et cyclohexyle sont les plus préférés.

5. Utilisation de cristaux dont la longueur dans une dimension est au moins deux fois la longueur dans toute autre direction, d'un dérivé de diurée à base d'hydrazine de formule
R'HN-CO-NH-NH-CO-NHR
dans laquelle R et R' sont indépendamment des groupes hydrocarbyle non substitués ou substitués, avec une longueur moyenne des cristaux de 0,1 à 300 micromètres, comme modificateur de rhéologie.

6. Composition de résine thixotrope selon la revendication 1, dans laquelle le dérivé de diurée à base d'hydrazine est présent dans une quantité concentrée de 0,1 à 50 % en poids, par rapport au poids de la composition.

7. Utilisation d'une résine selon la revendication 5 dans le procédé de préparation de compositions de revêtement ou adhésives.

8. Procédé de préparation d'une composition de résine thixotrope comprenant un modificateur de rhéologie dérivé de diurée à base d'hydrazine, impliquant les étapes consistant à i) faire réagir au moins un composé à fonctionnalité mono-isocyanate de formule R.NCO, où R a la signification donnée dans les revendications 1 à 4, avec une hydrazine pour former un dérivé de diurée à base d'hydrazine et ii) combiner le dérivé de diurée à base d'hydrazine avec une résine, pour obtenir des cristaux de type aiguille du dérivé de diurée à base d'hydrazine avec une longueur d'onde de 0,1 à 300, qui sont inclus dans la résine
la résine étant essentiellement dépourvue de formaldéhyde ou de composés qui génèrent du formaldéhyde, dans la mesure où il n'y a pas assez de formaldéhyde pour consommer complètement le dérivé de diurée à base d'hydrazine.

9. Procédé selon la revendication 8, dans lequel l'isocyanate et l'hydrazine réagissent en présence de la résine de telle sorte que les cristaux de type aiguille inclus du dérivé de diurée à base d'hydrazine sont obtenus en une seule étape.

10. Procédé selon la revendication 8 ou 9, dans lequel un ou plusieurs modificateurs de croissance cristalline sont utilisés pour réduire la longueur moyenne des cristaux à 0,1 à 100 micromètres.

11. Procédé pour durcir une composition de revêtement ou adhésive comprenant des cristaux dont la longueur dans une dimension est au moins deux fois la longueur dans toute autre direction, d'un modificateur de rhéologie dérivé de diurée à base d'hydrazine de formule
R'HN-CO-NH-NH-CO-NHR
dans laquelle R et R' sont indépendamment des groupes hydrocarbyle non substitués ou substitués, avec une longueur moyenne des cristaux de 0,1 à 300 micromètres, procédé dans lequel des conditions de séchage et/ou cuisson forcées sont appliquées.

12. Utilisation selon la revendication 5, dans laquelle les cristaux de dérivé de diurée à base d'hydrazine sont utilisés comme agent de contrôle de coulure ou agent anti-sédimentation dans des compositions de revêtement et/ou adhésives.

13. Procédé selon la revendication 8, dans lequel la longueur moyenne des cristaux de type aiguille du dérivé de diurée à base d'hydrazine est de 0,5 à 100 micromètres.
